(19) Europäisches Patentamt
European Patent Office
Office européen des brevets

(11) Veröffentlichungsnummer: **0 453 638 A1**

# (12) EUROPÄISCHE PATENTANMELDUNG

(21) Anmeldenummer: **90122388.3**

(22) Anmeldetag: **23.11.90**

(51) Int. Cl.$^5$: **C07D 233/32**, C07D 239/10, C07D 243/04, C07D 245/02

(30) Priorität: **02.04.90 DE 4010532**

(43) Veröffentlichungstag der Anmeldung:
**30.10.91 Patentblatt 91/44**

(84) Benannte Vertragsstaaten:
**AT BE CH DE DK ES FR GB IT LI NL SE**

(71) Anmelder: **RÖHM GMBH**
**Kirschenallee**
**W-6100 Darmstadt(DE)**

(72) Erfinder: **Hohage, Heinz, Dr.**
**In der Wildnis 3**
**W-6109 Mühltal(DE)**
Erfinder: **Neeb, Rolf**
**An der Römerstrasse 12**
**W-6102 Pfungstadt(DE)**

## (54) Verfahren zur Herstellung von endständig Heterocyclus-substituierten Acryl- und Methacrylsäurealkylestern.

(57) Es wird ein Verfahren zur Herstellung von (Meth)Acrylatestern der Formel I

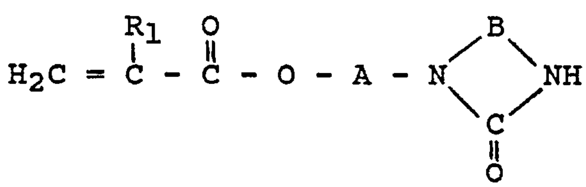

I

mit $R_1$ = H oder $CH_3$ und mit A und B unverzweigten oder verzweigten Alkylengruppen mit 2 bis 5 C-Atomen, durch Umsetzung von (Meth)Acrylestern der Formel II

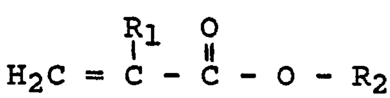

II

mit $R_2$ = Alkylrest von insbesondere 1 bis 4 C-Atomen und Alkoholen der Formel III

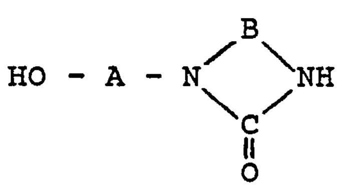

III

in Gegenwart von Dialkylzinnoxid als Katalysator beschrieben. Nach der Umsetzung kann der Katalysator durch Zusatz von Wasser aus dem Reaktionsgemisch abgetrennt, isoliert und erneut eingesetzt werden.

EP 0 453 638 A1

## Gebiet der Erfindung

Die vorliegende Erfindung betrifft ein verbessertes Verfahren zur Herstellung von Acryl- oder Methacrylsäureestern der Formel

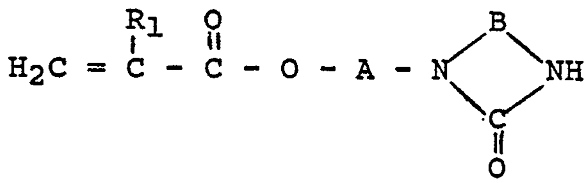

I

in der $R_1$ für Wasserstoff oder eine Methylgruppe steht und A und B unverzweigte oder verzweigte Alkylengruppen mit 2 bis 5 C-Atomen bedeuten.

## Stand der Technik

Verbindungen der Formel I lassen sich nach dem in der US-Patentschrift 2 871 223 beschriebenen Verfahren durch Umsetzung von Acryl- oder Methacrylsäurechlorid mit Hydroxialkylimidazolidin-2-onen in Gegenwart tertiärer Stickstoffbasen, wobei stöchiometrische Mengen der Hydrochloride der tertiären Stickstoffbasen mit anfallen, erhalten.
Bei dem aus der EP 0 236 994 A1 bekannten Verfahren zur Herstellung von Acryl- und Methacrylestern der Formel I werden Acryl- oder Methacrylsäureester mit 1-(Hydroxialkyl)-imidazolidin-2-onen in Gegenwart von Titanalkoholaten oder Chelatverbindungen der Metalle Titan, Zirkonium, Eisen und Zink mit 1,3-Dicarbonylverbindungen als Umesterungskatalysatoren umgesetzt.
In der Regel muß aus den Ansätzen nach Beendigung der Umsetzung der Metallkatalysator abgetrennt werden. Dies geschieht vorteilhaft, so z.B. bei Verwendung von Tetraalkyltitanaten, durch Zusetzen von Wasser, wodurch Metall(hydr)oxide, wie z.B. $TiO_2$ entstehen, die beispielsweise durch Filtrieren oder Zentrifugieren abgetrennt werden. Die hydrolysierten Umesterungskatalysatoren können nach Abtrennung nicht mehr als solche erneut eingesetzt werden.

## Aufgabe und Lösung

Der Erfindung lag die Aufgabe zugrunde, ein katalytisches Verfahren zur Herstellung von Acryl- oder Methacrylsäureestern der Formel I durch Alkoholyse von (Meth)Acrylsäurealkylestern mit Hydroxialkylimidazolidin-2-onen zu finden, bei denen der verwendete Katalysator aus dem Reaktionsgemisch abgetrennt und als solcher wieder eingesetzt werden kann.
Es wurde ein Verfahren gefunden, nach dem (Meth)Acrylester der Formel I

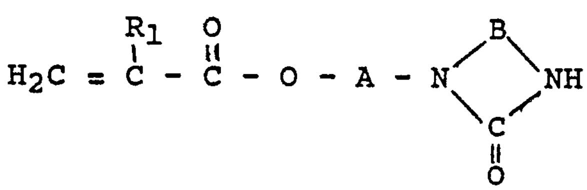

I

in der $R_1$ für Wasserstoff oder eine Methylgruppe steht und A und B unverzweigte oder verzweigte Alkylengruppen mit 2 bis 5 C-Atomen bedeuten, hergestelt werden, durch Umsetzung eines Acrylsäureesters oder Methacrylsäureesters der Formel II

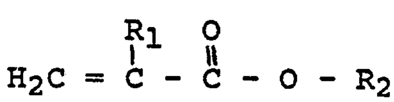

II

in der $R_1$ oben angegebene Bedeutung und $R_2$ die Bedeutung eines Alkylrestes mit 1 bis 4 C-Atomen haben, mit einer heterocyclischen Verbindung der Formel III

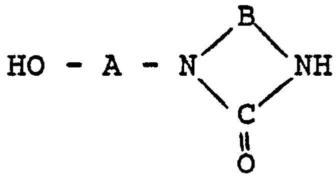

III

das dadurch gekennzeichnet ist, daß die Umsetzung eines Esters entsprechend der Formel II mit einer heterocyclischen Verbindung der Formel III zu einem Acryl- oder Methacrylester der Formel I in Gegenwart von Dialkylzinnoxid als Katalysator durchgeführt wird.

Vorteil dieses Verfahrens ist, daß nach Zugabe von Wasser, der Katalysator, das Dialkylzinnoxid, praktisch quantitativ aus dem Reaktionsgemisch ausgefällt und z.B. durch Filtration abgetrennt und dann wieder erneut als Katalysator eingesetzt werden kann.

Verbindungen der Formel I sind wertvolle Comonomere und werden beispielsweise bei der Herstellung von Polymerdispersionen aus Vinylmonomeren eingesetzt, die vor allem als Bindemittel z.B. in Lacken, oder als Lederhilfsmittel Verwendung finden. Comonomere der Formel I verleihen Copolymerisaten eine gewünschte Hydrophilie und können in wärmehärtbaren Harzen mit ihrer Imidgruppe als Formaldehydfänger fungieren.

Der Erfolg des erfindungsgemäßen Verfahrens ist überraschend, da aufgrund der Bifunktionalitäten von I und von II bei deren Umsetzung mit weiteren Reaktionen, wie Additionsreaktionen analog einer Michaeladdition an die Doppelbindung, oder mit einer Amidbildung durch Reaktion des Acryl- oder Methacrylesters I mit der NH-Gruppierung einer Verbindung der Formel II zu rechnen war. Die Umsetzung von Acryl- und Methacrylestern mit primären und sekundären Aminen unter dem Einfluß von Dialkylzinnoxid-Katalysatoren zu N-substituierten Acryl- und Methacrylamiden ist aus der DE-PS 28 16 516 C2 bekannt.

Nach dem erfindungsgemäßen Verfahren werden Verfahrensprodukte I erhalten, die ohne preislich und qualitativ belastende Abtrennverfahren, unmittelbar für die Verwendung als Comonomere, vor allem bei der Herstellung von Dispersionspolymerisaten, eingesetzt werden können, und die weiter vorteilhaft nicht mehr oder höchstens nur noch spurenweise die Schwermetallkomponente Zinn, vor allem als Dialkylzinnoxid, enthalten.

Durchführung der Erfindung

Für die Herstellung der Verbindungen I nach dem erfindungsgemäßen Verfahren werden Acryl- oder Methacrylsäureester der Formel II verwendet, in denen $R_2$ vor allem ein Alkylrest mit 1 bis 4 Kohlenstoffatomen bedeutet. Beispielsweise seien dazu Propylacrylat, n-Butylacrylat, Ethylmethacrylat, i-Propylmethacrylat, i-Butylmethacrylat, n-Butylmethacrylat und vor allem Methylmethacrylat genannt.

Als Ausgangsstoffe der Formel III kommen solche Verbindungen in Frage, in denen A oder B eine verzweigte oder unverzweigte Alkylengruppe mit 2 bis 5 Kohlenstoffatomen darstellt, z.B. $-(CH_2)_2-$, $-(CH_2)_3-$, $-(CH_2)_4-$, $-CH_2CH(CH_3)CH_2-$, $-CH_2C(CH_3)_2CH_2-$. Die Ringgliederzahl des Heterocyclus beträgt vorzugsweise 5 und 6. Besonders vorteilhaft wird als Verbindung III 1-(2-Hydroxiethyl)-imidazolidin-2-on, das z.B. nach der US-PS 3 254 075 gut und technisch aus Aminoethylethanolamin und Harnstoff herstellbar ist, verwendet.

In der DE-PS 10 05 947 werden Verfahren zur Herstellung von Estern beschrieben, wobei zur Durchführung verschiedener Esterherstellungs-Reaktionen Organozinnverbindungen als Katalysatoren verwendet werden. Die für die erfindungsgemäße Herstellung der Acryl- oder Methacrylester der Formel I vorteilhaften Dialkylzinnoxide sind Verbindungen der Formel IV

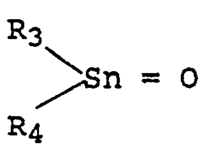

IV

wobei $R_3$ und $R_4$ Alkylreste mit beispielsweise 1 bis 12 C-Atomen in jedem Alkylrest bedeuten. Ein

vorteilhaft und vorzugsweise verwendetes Dialkylzinnoxid ist das als Handelsprodukt erhältliche Dibutylzinnoxid.

Die Umsetzung von Acrylestern und/oder Methacrylestern der Formel II mit den Alkoholen der Formel III (Alkoholyse) wird bei Temperaturen zwischen 30 und 180 Grad C, vor allem zwischen 50 und 130 Grad C, in Gegenwart von 0,01 bis 10 Gew.-%, vor allem in Gegenwart von 0,1 bis 10 Gew.-% und insbesondere von 0,2 bis 5 Gew.-%, berechnet auf das Gewicht des Reaktionsgemisches, des Katalysators durchgeführt.

Formal reagieren äquimolare Mengen der Reaktionspartner II und III zu den gewünschten Endprodukten III. In der Praxis hat es sich jedoch als zweckmäßig erwiesen, während der Umsetzung die Ausgangsester II stets im Überschuß zu halten. Sie werden in Mengen von 1 bis 20, vorzugsweise 2 bis 10, insbesondere 3 bis 6 Mol pro Mol III eingesetzt.

Zur Vermeidung von Polymerisationsverlusten ist es zweckmäßig, die Umsetzung und Aufarbeitung des Reaktionsgemisches in Gegenwart von Polymerisationsinhibitoren wie z.B. Phenothiazin, Hydrochinonmonomethylether und insbesondere Sauerstoff, durchzuführen.

Die Umsetzung kann unter Normaldruck, Unter- oder Überdruck ablaufen. Sie kann diskontinuierlich oder kontinuierlich erfolgen. Die Ausgangsstoffe II und III werden beispielsweise gemeinsam in Gegenwart von Dialkylzinnoxid zum Sieden erhitzt und dabei kontinuierlich der abgespaltene Alkohol $R_2OH$, gegebenenfalls in Form seines Azeotrops mit dem Ester II abdestilliert. Je nach Reaktionstemperatur, Katalysator bzw. Katalysatormenge liegen die Umsetzungszeiten im Bereich von ca. 1 bis 7 Stunden. Es ist auch möglich, die Umsetzung in Gegenwart eines inerten Lösungsmittels, z .B. Toluol oder Cyclohexan, durchzuführen.

Nach Beendigung der Umsetzung kann überschüssiger monomerer Ester II vollkommen oder teilweise durch Abdestillieren entfernt werden.

Die Abtrennung des Dialkylzinnoxid-Katalysators wird durch Zugabe von Wasser zum Reaktionsgemisch, das den überschüssigen Ester II noch teilweise enthalten kann, erreicht. Dazu wird Wasser in Mengen von 1 bis 200 Gew.-%, vorzugsweise in Mengen von 10 bis 150 Gew.-%, vor allem in Mengen von 20 bis 100 Gew.-%, bezogen auf das Gewicht des erhaltenen Reaktionsgemisches, vorteilhaft nach dem Abkühlen desselben, insbesondere bei Temperaturen von 10 bis 50 Grad C, zugesetzt.
Das Dialkylzinnoxid fällt dabei in abfiltrierbarer Form an und kann dann, gegebenenfalls nach Trocknung, wieder in weiteren Alkoholyseansätzen eingesetzt werden.
Ein bevorzugtes Umsetzungsprodukt ist ein solches, das aus Methylmethacrylat und 1-(2-Hydroxiethyl)-imidazolidin-2-on entsteht und so der Formel I mit $R_1 = CH_3$, $A = -(CH_2)_2-$ und $B = -(CH_2)_2-$ entspricht.

Für die Herstellung von Polymerdispersionen können etwa 5 bis 95 Gew.-%ige wäßrige Löungen der Produkte I weiterverwendet werden.

BEISPIEL

N-(2-Methacryloyloxyethyl)ethylenharnstoff

In einer Alkoholyseapparatur werden 350,0 g (2,7 mol) 1-(2-Hydroxiethyl)-imidazolidin-2-on, auch als 1-(2-Hydroxiethyl)ethylenharnstoff (HEEH) bezeichnet, 1000,0 g (10 mol) Methacrylsäuremethylester (MMA), 0,5 g Hydrochinonmonomethylether (400 ppm ber. auf MMA), 0,1 g Phenothiazin (100 ppm ber. auf MMA), 0,005 g Tempol $^{R}$ (5 ppm ber. auf MMA) vorgelegt und unter Luftdurchleiten innerhalb 2 Stunden entwässert (HEEH enthält bis 10 % Wasser). Anschließend wird abgekühlt, 13,4 g Dibutylzinnoxid (1,0 % ber. auf Ansatz) zugegeben und innerhalb von 2 Stunden MMA/$CH_3OH$-Azeotrop (Übergangstemperatur 75 - 82 Grad C, Rücklaufverhältnis 15 : 1) bis 98 % Umsatz abdestilliert. Danach wird bei 25 mbar, 60 Grad C Badtemperatur das überschüssige MMA abdestilliert. Man erhält 531,0 g Methacryloyloxyethylethylenharnstoff roh (gelbliche Kristalle). Zur Entfernung des Sn-Katalysators löst man das Rohprodukt in 495,0 g Wasser und filtriert über einen Seitz S 300-Tiefenfilter. Der in quantitativer Ausbeute erhaltene Ester enthält als Verunreinigung 1,6 % Edukt, 1,7 % Vernetzer und < 0,005 % Zinn.

## Patentansprüche

1. Verfahren zur Herstellung von (Meth)Acrylestern der Formel I

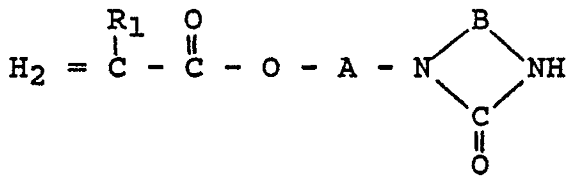

I

in der $R_1$ für Wasserstoff oder eine Methylgruppe steht und A und B unverzweigte oder verzweigte Alkylengruppen mit 2 bis 5 C-Atomen bedeuten, durch Umsetzung eines Acrylsäureesters oder Methacrylsäureesters der Formel II

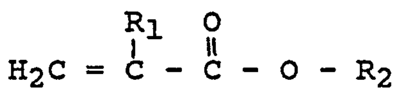

II

in der $R_1$ oben angegebene Bedeutung und $R_2$ die Bedeutung eines Alkylrestes mit 1 bis 4 C-Atomen haben, mit einer heterocyclischen Verbindung der Formel III

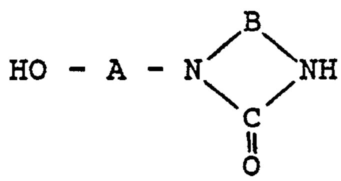

III

dadurch gekennzeichnet, daß die Umsetzung von II mit III zu einem Acryl- oder Methacrylester der Formel I in Gegenwart von Dialkylzinnoxid als Katalysator durchgeführt wird.

2. Verfahren nach Anspruch 1, dadurch gekennzeichnet, daß die Umsetzung von Acryl- oder Methacrylestern der Formel II mit 1-(2-Hydroxiethyl)-imidazolidin-2-on durchgeführt wird.

3. Verfahren nach den Ansprüchen 1 und 2, dadurch gekennzeichnet, daß Methylmethacrylat als Verbindung der Formel II mit einem Alkohol der Verbindung III umgesetzt wird.

4. Verfahren nach den Ansprüchen 1 bis 3, dadurch gekennzeichnet, daß der Dialkylzinnoxid-Katalysator nach der Alkoholyse-Reaktion und gegebenenfalls nach Abtrennung von überschüssigem Acryl- bzw. Methacrylester der Formel II, durch Zusatz von Wasser aus dem Reaktionsgemisch abgeschieden und davon abgetrennt wird.

5. Verfahren nach den Ansprüchen 1 bis 3, dadurch gekennzeichnet, daß ein nach Anspruch 4 abgetrennter Dialkylzinnoxid-Katalysator als Umsetzungskatalysator eingesetzt wird.

Europäisches Patentamt

# EUROPÄISCHER RECHERCHENBERICHT

Nummer der Anmeldung

EP 90 12 2388

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 124 713 (NATIONAL STARCH AND CHEMICAL CORP.) | | C 07 D 233/32<br>C 07 D 239/10<br>C 07 D 243/04<br>C 07 D 245/02 |
| A | DE-A-2 334 827 (CIBA-GEIGY AG) | | |
| A | DE-A-2 334 826 (CIBA-GEIGY AG) | | |
| D,A | EP-A-0 236 994 (BASF AG) | | |
| D,A | US-A-2 871 223 (ELINOR M. HANKINS AND SIDNEY MELAMED (ROHM & HAAS CO.)) | | |
| D,A | DE-A-2 816 516 (RÖHM GmbH) | | |
| D,A | DE-B-1 005 947 (THE B.F. GOODRICH CO.) | | |
| E | EP-A-0 433 135 (ATOCHEM)<br>* Das ganze Dokument * | 1-4 | |

| RECHERCHIERTE SACHGEBIETE (Int. Cl.5) |
|---|
| C 07 D 233/00<br>C 07 D 239/00<br>C 07 D 243/00<br>C 07 D 245/00 |

Der vorliegende Recherchenbericht wurde für alle Patentansprüche erstellt

| Recherchenort | Abschlußdatum der Recherche | Prüfer |
|---|---|---|
| Den Haag | 10 Juli 91 | DE BUYSER I.A.F. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument